(19) Europäisches Patentamt
European Patent Office
Office européen des brevets

(11) Numéro de publication: **0 024 219**
**B1**

# (12) FASCICULE DE BREVET EUROPEEN

(45) Date de publication du fascicule du brevet: **21.04.82**

(21) Numéro de dépôt: **80401044.5**

(22) Date de dépôt: **11.07.80**

(51) Int. Cl.³: **C 07 C 179/10,**
C 07 C 179/12,
C 07 C 178/00

(54) **Procédé de fabrication de solutions diluées stables de peracides carboxyliques aliphatiques et solutions obtenues.**

(30) Priorité: **01.08.79 FR 7919761**

(43) Date de publication de la demande:
**25.02.81 Bulletin 81/8**

(45) Mention de la délivrance du brevet:
**21.04.82 Bulletin 82/16**

(84) Etats contractants désignés:
**BE CH DE FR GB IT LI NL SE**

(56) Documents cités:
**FR - A - 2 309 531**
**FR - A - 2 321 301**
**FR - A - 2 321 302**

(73) Titulaire: **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cedex 07 (FR)**

(72) Inventeur: **Crommelynck, François**
**Route de Narbonne**
**F-38000 Saint-Martin-le-Vinoux (FR)**
Inventeur: **Granger, Michel**
**24, place Mathias**
**F-71100 Châlon-sur-Saône (FR)**
Inventeur: **Rio, Michel**
**3, avenue des Croisades**
**F-78190 Elancourt (FR)**
Inventeur: **Tourdot, Jacques**
**2, rue du Général Henrys**
**F-75017 Paris (FR)**

(74) Mandataire: **Bouton Neuvy, Liliane et al,**
**L'Air liquide, Société Anonyme pour L'Etude et L'Exploitation des Procédés Georges Claude 75, Quai d'Orsay**
**F-75321 Paris Cedex 07 (FR)**

**EP 0 024 219 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé de fabrication de solutions diluées stables de peracides carboxyliques aliphatiques et solutions obtenues

La présente invention concerne un procédé de fabrication de solutions diluées stables peracides carboxyliques aliphatiques, notamment d'acide monoperacétique.

On sait que les solutions d'acide monoperacétique, préparées à partir de peroxyde d'hydrogène et d'acide acétique, obéissent à une loi d'équilibre dont l'équation peut être schématisée de la manière suivante:

$$CH_3{-}CO_2H + H_2O_2 \underset{2}{\overset{1}{\rightleftarrows}} CH_3 - CO_3H + H_2O$$

La cinétique d'évolution de ce système est relativement lente; elle est accélérée par des acides forts tels que l'acide sulfurique $SO_4H_2$ ou l'acide phosphorique $PO_4H_3$ — qui jouent, en l'occurence, le rôle de catalyseur — la présence de ces acides forts permet au système d'évoluer plus rapidement jusqu'à son état d'équilibre.

Toutefois, il est connu qu'une fois parvenu à son état d'équilibre, le système perd lentement l'oxygène actif qu'il contient; cette perte se traduisant, par une lente décroissance de la teneur en peracide et en peroxyde d'hydrogène. On tend à pallier ce phénomène par addition de stabilisant.

Tous ces phénomènes ont notamment été mis en évidence par F. K. Greenspan (J. Amer. Chem. Soc., 1946, 68, 907). L'auteur a observé par ailleurs que les solutions concentrées possèdent une relative stabilité donnant ainsi la possibilité de commercialiser l'acide peracétique à des concentrations élevées dans des solutions stabilisées. Actuellement, l'acide monoperacétique est couramment commercialisé à des concentrations de 35 à 45%.

Cependant, d'autres utilisations de l'acide monoperacétique, telles son emploi dans la désinfection et la stérilisation, dans les domaines médicaux et alimentaires réclament des solutions diluées d'acides percarboxyliques aliphatiques stables dans le temps.

Différents procédés ont été proposés en vue de l'obtention de solutions d'acide peracétique dilué, de concentration comprise entre 0,5 et 20%.

Le brevet français 1.352.479 de FMC Corporation a trait à un procédé continu, par réaction d'un acide aliphatique inférieur avec du peroxyde d'hydrogène à raison de 0,3 à 5 moles de peroxyde d'hydrogène par mole d'acide, en présence de 5 à 20% d'un acide forte entraineur d'eau. Quant au brevet français 1.452.484, émanant de la même Société, il concerne un procédé de production de solutions aqueuses étendues d'acide peracétique, ayant une concentration de 0,5 à 7% en poids en acide peracétique, un pH de 5,4 à 7 et exemptes de peroxyde diacétyle.

Ces procédés de préparation d'acide peracétique ont pour but de séparer l'acide peracétique après sa formation dans le mélange réactionnel, soit en raison des réactions secondaires provoquées par l'acide sulfurique, l'$H_2O_2$ et l'acide acétique dans les réactions d'époxydation, soit en raison des problèmes de sécurité soulevés par la présence du peroxyde de diacétyle dans les opérations de blanchiment.

Toutefois, pour d'autres utilisations de l'acide peracétique, telles que son emploi pour la désinfection et la stérilisation, il n'est par nécessaire de le séparer du mélange réactionnel et les solutions d'acide peracétique, obtenues avant sa séparation, selon les procédés décrits dans les brevets précédents, pourraient être utilisées pour la stérilisation et la désinfection. Il en est de même pour les solutions d'acide peracétique obtenues par Grennspan dès 1946.

Plus récemment, des concentrés contenant 0,5 à 20% de peracide à 2 ou 3 atomes de carbone et/ou d'acide monocarboxylique aliphatique correspondant, 25 à 40% de peroxyde d'hydrogène, en outre 0,05 à 5% en poids de mouillant anionique sous forme d'alkylbenzènesulfonate, d'alkylsulfate et/ou d'alcanesulfonate, et le complément en eau, ont fait l'objet de la demande de brevet d'invention 2.321.301; tandis que les concentrés contenant 0,25 à 10% d'acide phosphonique ou de sels acides hydrosolubles correspondants sont décrits dans la demande de brevet 2.321.302, l'un et l'autre au nom de la Société Henkel.

De toute manière si l'on envisage de fabriquer industriellement et même de commercialiser des solutions d'acide peracétique à faibles concentrations, il apparait nécessaire que ces solutions répondent à deux critères fondamentaux:

— La fabrication industrielle de ces solutions doit être réalisée dans un délai qui n'impose pas un stockage rédhibitoire, sur le plan économique, des mélanges réactionnels, pour obtenir la concentration souhaitée en acide peracétique.

Sur ce point, il convient que le mélange réactionnel atteinge au moins 90% de la concentration maximale obtenue à l'équilibre dans un délai inférieur ou égal à 48 heures.

— La stabilité de la solution d'acide peracétique, à la température ambiante, doit être telle que la concentration en acide peracétique après 12 mois de stockage, ne soit pas inférieure à 90% de la concentration maximale atteinte par le mélange réactionnel, cette concentration maximale pouvant être d'ailleurs différente de la concentration nominale du mélange.

En d'autres termes, une solution d'acide peracétique commercialisée à une concentration nominale de 5% par exemple, devrait avoir une concentration de 5% au moment de sa commercialisation, de 5,55% à sa concentration maximale et de 5% après 12 mois de stockage à la température ambiante.

Il a été trouvé un procédé qui permet de répondre aux exigences industrielles, en résolvant les difficultés inhérentes à une fabrication rapide et à une conservation prolongée de solutions diluées de peracides carboxyliques aliphatiques à titre stable dans le temps.

Selon l'invention, dans un premier stade on prépare une solution concentrée de peracide aliphatique à partir de l'acide ou de l'anhydride correspondant et de peroxyde d'hydrogène concentré, en présence de la quantité minimale d'une catalyseur acide fort nécessaire à l'obtention de l'équilibre du système dans un délai maximal de 48 heures; et en ce que dans un second stade on dilue la dite solution concentrée de peracide aliphatique avec une solution contenant au moins un des réactifs pour amener la concentration en peracide aliphatique à la concentration nominale du mélange. Cette concentration peut par exemple être de 5% en peracide aliphatique.

Le procédé permet d'obtenir dans un délai compatible avec une fabrication industrielle, une solution concentrée de peracide aliphatique contenant le minimum de catalyseur acide et, si nécessaire, en amenant rapidement le système à l'équilibre en le portant à une température légèrement supérieure à la température ambiante.

Dans le second stade, la concentration de la solution de dilution est telle qu'une fois la concentration en peracide carboxylique aliphatique amenée à la valeur nominale fixée, la solution ainsi obtenue contienne une quantité de réactif permettant de faire évoluer le mélange vers la formation de peracide carboxylique.

Le constituant réactionnel présent dans la solution de dilution est soit le peroxyde d'hydrogène soit l'acide carboxylique de départ, seuls ou en mélange. De préférence la solution de dilution est une solution de peroxyde d'hydrogène.

La quantité du constituant réactionnel, tel le peroxyde d'hydrogène, contenue dans la solution diluée de peracide aliphatique est telle que, pendant la conservation de la solution diluée à la température ambiante, la concentration maximale en peracide soit au plus de 10% supérieure à la concentration nominale du peracide (par exemple 5,5% en poids) et que la concentration finale soit au moins égale à la concentration nominale du peracide aliphatique, la concentration nominale et initiale pouvant être la même (par exemple 5% en poids).

La dilution de la solution concentrée par un des réactifs ou le mélange de ceux-ci, notamment par le peroxyde d'hydrogène permet d'abaisser la concentration du catalyseur acide, ce qui ralentit considérablement la cinétique du système. D'autre part, la concentration du ou des réactifs de dilution est choisie de telle manière qu'une fois la dilution effectuée, le système ne soit plus à l'équilibre, mais tende à évoluer dans le sens de la formation de peracide aliphatique à une vitesse excessivement lente. L'évolution du système vers la formation de peracide, bien que très lente, permet de compenser la décomposition catalytique pendant plusieurs mois de conservation.

Dans le premier stade de fabrication on prépare avantageusement la solution concentrée de peracide aliphatique à partir de l'acide ou de l'anhydride correspondant, de peroxyde d'hydrogène à concentration comprise entre 60 et 90% en poids, de préférence 70 à 85%. Dans le second stade, on dilue avantageusement la solution concentrée de peracide aliphatique par du peroxyde d'hydrogène à concentration comprise entre 40 et 70%, de préférence entre 40 et 50% en poids.

Selon le procédé de l'invention des solutions diluées de peracide carboxylique aliphatique peuvent être préparées à partir d'un acide monocarboxylique à 2 ou 3 atomes de carbone ou d'un acide dicarboxylique contenant de 3 à 5 atomes de carbone dans la molécule, en particulier l'acide acétique.

Le catalyseur acide fort utilisé dans la préparation de la solution concentrée de peracide est un acide minéral fort, tel l'acide sulfurique ou orthophosphorique ou un acide organominéral. On emploie avantageusement ce catalyseur en réalisant la réaction en présence de 0,1 à 5% en poids de catalyseur par rapport au poids du mélange réactionnel, préférentiellement cette quantité catalytique sera de 0,2 à 0,5% en poids par rapport au poids du mélange réactionnel.

Selon une variante, la solution étendue de peracide aliphatique peut contenir un stabilisant, éventuellement introduit dans la solution concentrée de peracide aliphatique. On connait, de nombreux produits chimiques, utilisés seuls ou en mélange, qui possèdent des propriétés stabilisantes compatibles avec le procédé, à savoir, notamment: l'acide dipicolinique, le stannate de sodium, le salicylate de sodium, l'hydroquinone et le tertiobutylhydroxytoluène (BHT).

Selon l'invention, on obtient aisément et dans de très bonnes conditions industrielles des solutions diluées contenant entre 1 et 20% en poids de peracide carboxylique, en particulier d'acide monoperacétique. Préférentiellement, la concentration des solutions préparées contiennent de 2 à 5% en poids de peracide carboxylique.

L'obtention de solutions faiblement concentrées en acide peracétique selon l'invention apporte par conséquent un facteur de sécurité supplémentaire quant à la garantie de la concentration nominale en acide peracétique pour un produit industriel destiné à être utilisé par exemple pour des opérations de désinfection ou de stérilisation dans les domaines médicaux ou alimentaires.

Dans ce cas, les solutions d'acide peracétique dilué sont parfois additionnées de petites quantités d'agents tensio-actifs tels : alkyléthersulfates, détergents non ioniques (acides gras et résiniques) polyéthoxylés, alcools gras polyéthoxylés et les sels alcalines de leurs dérivés sulfatés, les sels alcalins des

dérivés sulfatés des alkylphénols polyéthoxylés, alcanolamides d'acides gras, esters d'acides gras de saccharose et de sucroglycérides.

Si l'on considère qu'un tel produit industriel doit pouvoir être utilisé, sans risque de perte d'efficacité, pendant une période du un an, ce facteur de sécurité est d'autant plus appréciable qu'il permet de compenser la décomposition catalytique de l'acide peracétique; or, dans le cadre d'une fabrication industrielle, les microtraces d'impuretés susceptibles d'avoir une action catalytique sur la décomposition de l'acide peracétique, peuvent avoir des origines diverses et sont parfois difficilement contrôlables.

Dans ces conditions, l'effet des stabilisants employés pour diminuer la cinétique de cette décomposition catalytique, peut être relativement fluctuant dans le courant. d'une fabrication industrielle. Cet aspect risque d'affecter les solutions diluées d'acide peracétique qui seraient vendues à leur état d'équilibre ou proche de l'équilibre et contenant la dose entière de catalyseur utilisé pour leur fabrication. D'autre part, l'élimination du catalyseur utilisé par des procédés de séparation physico-chimique augmenterait, sensiblement le prix de revient de telles solutions.

Il est donné ci-après des exemples qui illustrent l'invention à titre non limitatif.

Exemple 1

On prépare une solution concentrée d'acide peracétique en écoulant sous agitation 404 g de peroxyde d'hydrogène à 90% en poids dans un ballon en verre immergé dans un bain d'eau et contenant 590,25 g d'acide acétique additionné de 5 g d'acide sulfurique et de 0,75 g d'acide picolinique. On maintient le mélange entre 20 et 22°C, et la concentration en acide peracétique du mélange atteint 45% après 30 heures environ.

Le mélange a alors la composition suivante exprimée en pourcentage en poids.

| | % en poids |
|---|---|
| — Acide peracétique | 45 |
| — Acide acétique | 23,6 |
| — $H_2O_2$ | 16,1 |
| — $H_2O$ | 14,8 |
| — $SO_4H_2$ | 0,5 |
| — Acide dipicolinique | 750 ppm |

On dilue cette solution concentrée au dixième avec respectivement du peroxyde d'hydrogène à 30, 35, 40, 45 et 50% de façon à abaisser la concentration en acide peracétique de 45% à 4,5% d'une manière quasi instantanée.

On stocke les solutions ainsi obtenues à la température ambiante durant 12 mois; l'évolution de la teneur en $H_2O_2$ et en acide peracétique, en % en poids, est suivie dans le temps en soumettant à l'analyse des échantillons prélevés dans chaque solution. Les résultats sont rassemblés dans le tableau ici-dessous.

TABLEAU I

| Temps | $H_2O_2$ 30% $H_2O_2$ % | $H_2O_2$ 30% APA | $H_2O_2$ 35% $H_2O_2$ % | $H_2O_2$ 35% APA | $H_2O_2$ 40% $H_2O_2$ % | $H_2O_2$ 40% APA | $H_2O_2$ 45% $H_2O_2$ % | $H_2O_2$ 45% APA | $H_2O_2$ 50% $H_2O_2$ % | $H_2O_2$ 50% APA |
|---|---|---|---|---|---|---|---|---|---|---|
| –0 | 28,7 | 4,5 | 33 | 4,5 | 37,6 | 4,5 | 42,1 | 4,5 | 46,6 | 4,5 |
| –1 mois | 28,5 | 3,7 | 32,8 | 4,2 | 37,5 | 4,6 | 41,6 | 4,9 | 45,3 | 5,3 |
| –2 mois | 28,5 | 3,6 | 32,8 | 4,2 | 37,5 | 4,8 | 41,4 | 5,2 | 45 | 5,7 |
| –5 mois | 28,5 | 3,5 | 32,7 | 4,1 | 37,4 | 4,8 | 41,3 | 5,1 | 44,8 | 5,6 |
| –7 mois | 28,4 | 3,4 | 32,6 | 4 | 37,2 | 4,8 | 41,3 | 5 | 44,6 | 5,5 |
| –10 mois | 28,4 | 3,4 | 32,4 | 4 | 36,8 | 4,7 | 41,2 | 4,9 | 44,4 | 5,2 |
| –12 mois | 28,3 | 3,3 | 32,3 | 3,9 | 36,5 | 4,7 | 41,1 | 4,8 | 44,3 | 5,1 |

On constate, pour toutes les solutions, une décroissance continue de la teneur en $H_2O_2$ avec le temps. On constate également une décroissance continue de la teneur en acide peracétique dans le temps pour les solutions diluées avec $H_2O_2$ à 30 et & 35%. Par contre, pour les solutions diluées avec $H_2O_2$ à 40, 45 et 50%, on observe dans le temps une croissance, puis une décroissance de la teneur en acide peracétique.

Comme le montrent les résultats du tableau I, l'acroissement de la teneur en acide peracétique s'effectue pendant plusieurs mois de stockage à la température ambiante; or, les solutions étudiées contiennent 0,05% de $SO_4H_2$.

Les variations en Δ% de la concentration maximale et de la concentration finale par rapport à la concentration initiale, pour chaque solution, sont les suivantes:

TABLEAU II

| | Concentration maximale<br>„ initiale<br>Δ% | Concentration finale<br>„ initiale<br>Δ% |
|---|---|---|
| — $H_2O_2$ 40% | 6,7 | 4,4 |
| — $H_2O_2$ 40% | 15,6 | 6,6 |
| — $H_2O_2$ 50% | 26,7 | 13,6 |

On constante, d'après les résultats consignés dans le tableau II, que la solution d'acide péracétique obtenue par dilution avec $H_2O_2$ à 40%, présente la variation minimale de la concentration en acide peracétique durant la période de conservation de 12 mois; elle est d'autre part la seule à répondre au critère de stabilité défini precédémment; les solutions obtenues par dilution avec $H_2O_2$ à 45 et 50% présentent des variations de concentration supérieures à 10%.

Exemple 2

A titre de comparaison, on suit l'évolution d'une solution d'acide peracétique faiblement concentré, préparée de façon classique à partir d'acide acétique glacial et d'$H_2O_2$ en présence d'un catalyseur acide fort et additionnée d'un stabilisant. On prépare cette solution à partir de 56 g d'acide acétique glacial additionnés de 1,04 g de $SO_4H_2$, mélangés avec 662,5 g d'$H_2O_2$ à 70% et 321,5 g d'eau désoinisée. Le mélange obtenu contient 0,1% de $SO_4H_2$; 75 ppm d'acide dipicolinique y sont rajoutés.

On suit l'évolution dans le temps de la teneur en acide peracétique en soumettant à l'analyse des échantillons du mélange conservé à la température ambiante. Les résultats sont rassemblés dans le tableau III.

TABLEAU III

| Temps | Acide peracétique |
|---|---|
| 8 jours | 2;3% |
| 15 „ | 3,2% |
| 21 „ | 3,5% |
| 30 „ | 3,7% |

Les résultats du tableau III montrent qu'à la température ambiante et avec 0,1% de $SO_4H_2$, on obtient 92% de l'équilibre après 21 jours et 97% de l'équilibre après 30 jours. Ces résultats mettent en évidence l'écart important qui existe entre la cinétique des solutions d'acide peracétique dilué et celle des solutions d'acide peracétique concentré. La cinétique est également dépendante de la concentration en catalyseur, acide fort ($SO_4H_2$ par exemple) et de la température.

Exemple 3

Etude de l'influence de la concentration en catalyseur et de la température.

On prépare une solution d'acide peracétique, selon la même méthode que précédemment, à partir d'acide acétique glacial et de $H_2O_2$ à 70% en poids. Le mélange contient, au départ, 1,5 mole d'acide acétique glacial pour 1 mole d'$H_2O_2$ 100% et 75 ppm d'acide dipicolinique; plusieurs mélanges ont été réalises avec des concentrations croissantes de $SO_4H_2$: 0,1%, 0,3%, 0,5%, 0.75%, 1%. D'autre part deux séries de mélanges identiques sont conservés l'un à 20°C, l'autre à 30°C. On mesure pour chaque mélange, le temps au bout duquel la concentration en acide peracétique atteint 35% dans le mélange.

Les résultats sont rassemblés dans le tableau IV.

TABLEAU IV

Temps pour obtenir 35% d'acide peracétique dans le mélange

| Température de conservation du mélange | $SO_4H_2$% 0,1 | 0,3 | 0,5 | 0,75 | 1 |
|---|---|---|---|---|---|
| 20°C | 110 heures | 42 heures | 21 heures | 15 heures | 10 heures |
| 30°C | 48 " | 19 " | 9 " | 6,5 " | 5 " |

On constate, d'après le tableau 4 a) que la cinétique est sensiblement augmentée par un facteur 2 en passant de 20°C à 30°C, b) et qu'elle est approximativement multipliée par un facteur 2 de 0,3 à 0,5%, puis un facteur 2 de 0,5 à 1%, soit un facteur de 10 environ en passant de 0,1 à 1% de $SO_4H_2$.

D'après les résultats du tableau 4 il apparait possible de préparer par exemple une solution à 35% d'acide peracétique avec 0,1% de $SO_4H_2$ dans un délai de 48 heures, à condition de maintenir le mélange réactionnel à une température de 30°C.

La dilution de cette solution à 35% d'acide peracétique avec de l'$H_2O_2$ de concentration 42% permet d'obtenir par exemple une solution à 5% d'acide peracétique ne contenant que 0,014% de $SO_4H_2$ (dilution au septième).

**Revendications**

1. Procédé de fabrication de solution diluée stable de peracide carboxylique aliphatique en présence d'une faible quantité de catalyseur, caractérisé en ce que dans un premier stade on prépare une solution concentrée de peracide aliphatique à partir de l'acide ou de l'anhydride correspondant et de peroxyde d'hydrogène concentré, en présence de la quantité minimale d'un catalyseur acide fort nécessaire à l'obtention de l'équilibre du système dans un délai maximal de 48 heures; et en ce que dans un second stade on dilue la dite solution concentrée de peracide aliphatique avec une solution contenant au moins un des réactifs pour amener la concentration en peracide aliphatique à la concentration nominale du mélange.

2. Procédé de fabrication de solution diluée stable de peracide selon la revendication 1, caractérisé en ce que dans le second stade la concentration de la solution de dilution est telle qu'une fois la concentration en peracide carboxylique aliphatique amenée à la valeur nominale fixée, la solution ainsi obtenue contienne une quantité de réactif permettant de faire évoluer le mélange vers la formation de peracide carboxylique.

3. Procédé de fabrication de solution diluée stable de peracide selon la revendication 1, caractérisé en ce que le constituant réactionnel présent dans la solution de dilution est choisi parmi le peroxyde d'hydrogène et l'acide carboxylique seuls ou en mélange.

4. Procédé de fabrication de solution diluée stable de peracide selon la revendication 3, caractérisé en ce que la quantité du constituant réactionnel, tel de peroxyde d'hydrogène, contenu dans la solution diluée de peracide aliphatique soit telle que pendant le conservation de la solution diluée à la température ambiante, la concentration maximale en peracide soit au plus 10% supérieure à la concentration nominale du peracide et que la concentration finale soit au moins égale à la concentration nominale du peracide aliphatique.

5. Procédé de fabrication de solution diluée stable de peracide selon la revendication 1, caractérisé en ce que l'on introduit un stabilisnt dans la solution concentrée de peracide aliphatique.

6. Procédé de fabrication de solution diluée stable de peracide selon la revendication 1, caractérisé en ce que dans le premier stade le catalyseur acide fort utilisé est un acide minéral fort, tel l'acide sulfurique ou orthophosphorique, ou un acide organominéral, utilisé à raison de 0,1 à 5% en poids par rapport au mélange réactionnel, de préférence 0,2 à 0,5%.

7. Procédé de fabrication de solution diluée stable de peracide selon la revendication 1, caractérisé en ce que dans le premier stade l'acide carboxylique est choisi parmi les acides monocarboxyliques à 2 ou 3 atomes de carbone et les acides dicarboxyliques contenant de 3 à 5 atomes de carbone dans la molécule.

8. Procédé de fabrication de solution diluée stable de peracide selon la revendication 3, caractérisé en ce que dans le premier stade on prépare la solution concentrée de peracide aliphatique à partir de l'acide ou de l'anhydride correspondant, de peroxyde d'hydrogène à concentration comprise entre 60 et 90% en poids, de préférence 70 à 80%, et en ce que dans le second stade on dilue la solution concentrée de peracide aliphatique par du peroxyde d'hydrogène à concentration comprise entre 40 et 70%, de préférence entre 40 et 50%.

9. Solutions diluées stables de peracide carboxylique aliphatique obtenues par le procédé selon une quelconque des revendications 1 à 8.

## Claims

1. Method of manufacturing a stable dilute solution of an aliphatic carboxylic peracid in the presence of a small quantity of catalyst, characterised in that in a first stage there is prepared a concentrated aliphatic peracid solution from the corresponding acid or anhydride and from concentrated hydrogen peroxide, in the presence of the minimum quantity of strong acid catalyst necessary to obtain equilibrium of the system within a maximum time of 48 hours; and in that in a second stage said concentrated aliphatic peracid solution is diluted with a solution containing at least one of the reagents in order to bring the aliphatic peracid concentration to the nominal concentration of the mixture.

2. Method of manufacturing a stable dilute peracid solution according to claim 1, characterised in that in the second stage of concentration of the diluting solution is such that once the aliphatic carboxylic peracid concentration has been brought to the fixed nominal value, the solution thus obtained contains a quantity of reagent which allows the mixture to evolve to form carboxylic peracid.

3. Method of manufacturing a stable dilute peracid solution according to claim 1, characterised in that the reactive constituent present in the diluting solution is selected from hydrogen peroxide and carboxylic acid, alone or in admixture.

4. Method of manufacturing a stable dilute peracid solution according to claim 3, characterised in that the quantity of the reactive constituent, such as hydrogen peroxide, contained in the dilute solution of an aliphatic peracid is such that whilst the solution is being stored at the ambient temperature, the maximum peracid concentration is at the maximum 10% higher than the nominal concentration of the peracid, and that the final concentration is at least equal to the nominal concentration of the aliphatic peracid.

5. Method of manufacturing a stable dilute peracid solution according to claim 1, characterised in that a stabilizer is introduced into the concentrated solution of an aliphatic peracid.

6. Method of manufacturing a stable dilute peracid solution according to claim 1, characterised in that in the first stage the strong acid catalyst used is a strong mineral acid, such as sulphuric or orthophosphoric acid, or an organomineral acid, used at the rate of 0.1 to 5% by weight relative to the reactive mixture, preferably 0.2 to 0.5%.

7. Method of manufacturing a stable dilute peracid solution according to claim 1, characterised in that int he first stage the carboxylic acid is selected from monocarboxylic acids having 2 or 3 atoms of carbon and from dicarboxylic acids containing 3 to 5 atoms of carbon in the molecule.

8. Method of manufacturing a stable dilute peracid soution according to claim 3, characterised in that in the first stage the concentrated solution of an aliphatic peracid is prepared from the corresponding acid or anhydride, from hydrogen peroxide having a concentration of between 60 and 90% by weight, preferably 70 to 85%, and in that in the second stage the concentrated solution of an aliphatic peracid is diluted by hydrogen peroxide having a concentration of between 40 and 70%, preferably between 40 and 50%.

9. Stable dilute solutions of an aliphatic carboxylic peracid obtained by means of the method according to any one of claims 1 to 8.

## Patentansprüche

1. Verfahren zur Herstellung einer stabilen verdünnten Lösung von aliphatischer Percarbonsäure in Gegenwart einer geringen Katalysatormenge, dadurch gekennzeichnet, daß man in einer ersten Stufe eine konzentrierte Lösung von aliphatischer Persäure aus der Säure oder dem entsprechenden Anhydrid und konzentrierten Wasserstoffperoxid in Gegenwart der Mindestmenge eines stark sauren Katalysators, die erforderlich ist, um das Gleichgewicht des Systems in einer Maximalzeit von 48 Stunden zu erhalten, herstellt, und daß man in einer zweiten Stufe diese konzentrierte Lösung von aliphatischer Persäure mit einer wenigstens einen der Reaktionspartner enthaltenden Lösung verdünnt, um die Konzentration an aliphatischer Persäure auf die Nominalkonzentration des Gemisches zu bringen.

2. Verfahren zur Herstellung einer stabilen verdünnten Persäurelösung nach Anspruch 1, dadurch gekennzeichnet, daß in der zweiten Stufe die Konzentration der Verdünnungslösung derart ist, daß, wenn die Konzentration an aliphatischer Percarbonsäure einmal auf den festgelegten Nominalwert gebracht ist, die so erhaltene Lösung eine Reaktionspartnermenge enthält, die das Gemisch zur Bildung von Percarbonsäure umkippen läßt.

3. Verfahren zur Herstellung von stabilier verdünnter Persäurelösung nach Anspruch 1, dadurch gekennzeichnet, daß der in der Verdünnungslösung vorliegende reaktionsfähige Bestandteil unter Wasserstoffperoxid und der Carbonsäure allein oder im Gemisch miteinander ausgewählt wird.

4. Verfahren zur Herstellung einer stabilen verdünnten Persäurelösung nach Anspruch 3, dadurch gekennzeichnet, daß die Menge des reaktionsfähigen Bestandteils, wie des Wasserstoffperoxids, der in der verdünnten Lösung von aliphatischer Persäure enthalten ist, derart ist, daß, während die verdünnte Lösung auf Umgebungstemperatur gehalten wird, die Maximalkonzentration an Persäure höchstens 10% uber der Nominalkonzentration an Persäure liegt und daß die Endkonzentration wenigstens gleich der Nominalkonzentration der aliphatischen Persäure liegt.

5. Verfahren zur Herstellung einer stabilen verdünnten Persäurelösung nach Anspruch 1, dadurch gekennzeichnet, daß man in die konzentrierte Lösung von aliphatischer Persäure ein Stabilisierungsmittel einführt.

6. Verfahren zur Herstellung einer stabilen verdünnten Persäurelösung nach Anspruch 1, dadurch gekennzeichnet, daß der in der ersten Stufe verwendete stark saure Katalysator eine starke Mineralsäure, wie Schwefelsäure oder Orthophosphorsäure, oder eine Organomineralsäure ist, die in einer Menge von 0,1 bis 5 Gewichts-%, bezogen auf das Reaktionsgemisch, vorzugsweise in einer Menge von 0,2 bis 0,5 Gewichts-%, verwendet wird.

7. Verfahren zur Herstellung einer stabilen verdünnten Persäurelösung nach Anspruch 1, dadurch gekennzeichnet, daß in der ersten Stufe die Carbonsäure unter den Monocarbonsäuren mit zwei oder drei Kohlenstoffatomen und den Dicarbonsäuren mit drei bis fünf Kohlenstoffatomen im Molekül ausgewählt wird.

8. Verfahren zur Herstellung einer stabilen verdünnten Persäurelösung nach Anspruch 3, dadurch gekennzeichnet, daß man in der ersten Stufe die konzentrierte Lösung von aliphatischer Persäure aus der Säure oder dem entsprechenden Anhydrid und aus Wasserstoff-peroxid mit einer Konzentration zwischen 60 und 90 Gewichts-%, vorzugsweise 70 bis 85 Gewichts-%, herstellt und daß man in der zweiten Stufe die konzentrierte Lösung von aliphatischer Persäure mit Wasserstoffperoxid einer Konzentration zwischen 40 und 70, vorzugsweise zwischen 40 und 50 Gewichts-% verdünnt.

9. Stabile verdünnte Lösungen von aliphatischen Carbonsäuren, die nach dem Verfahren nach einem der Ansprüche 1 bis 8 erhalten wurden.